(19) Europäisches Patentamt
European Patent Office
Office européen des brevets

(11) Numéro de publication : **0 682 025 A1**

(12) **DEMANDE DE BREVET EUROPEEN**

(21) Numéro de dépôt : **95401014.6**

(22) Date de dépôt : **03.05.95**

(51) Int. Cl.[6] : **C07D 471/16,** C07D 487/16, A61K 31/435, A61K 31/415, // (C07D471/16, 235:00, 235:00, 235:00, 221:00), (C07D487/16, 235:00, 235:00, 235:00, 209:00)

(30) Priorité : **10.05.94 FR 9405715**

(43) Date de publication de la demande : **15.11.95 Bulletin 95/46**

(84) Etats contractants désignés : **AT BE CH DE DK ES FR GB GR IE IT LI LU NL PT SE**

(71) Demandeur : **SYNTHELABO**
**22, Avenue Galilée**
**F-92350 Le Plessis Robinson (FR)**

(72) Inventeur : **George, Pascal**
**19 rue des Quatre Vents**
**F-78730 St. Arnoult en Yvelines (FR)**
Inventeur : **Sevrin, Mireille**
**73 rue Raymond Losserand**
**F-75014 Paris (FR)**
Inventeur : **Peynot, Michel**
**2 rue des Marguerites**
**F-94240 L'Hay les Roses (FR)**

(74) Mandataire : **Ludwig, Jacques et al**
**SYNTHELABO,**
**Service Brevets,**
**B.P. 72**
**F-92352 Le Plessis Robinson Cédex (FR)**

(54) **Dérivés de 5,6-dihydro-4H-imidazo 2',1':2,3 imidazo- 4,5,1-ij quinoléine et de 4,5-dihydroimidazo 1,2-a pyrrolo- 1,2,3-cd benzimidazole, leur préparation et leur application et thérapeutique.**

(57) Composés répondant à la formule générale (I)

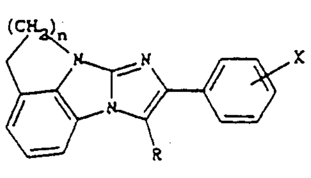

(I)

dans laquelle n représente 1 ou 2, X représente H ou un ou deux atomes ou groupes choisis parmi le fluor, le chlore et les groupes alkyles en $C_1$-$C_3$, alcoxy en $C_1$-$C_3$ et hydroxy, et R représente soit H soit un groupe de formule générale $-CH_2-CO_2-R_1$ (dans laquelle $R_1$ représente H ou un groupe alkyle en $C_1$-$C_6$), soit un groupe de formule générale $-CH_2-CO-NR_2R_3$ (dans laquelle $R_2$ et $R_3$ représentent chacun H ou un groupe alkyle en $C_1$-$C_3$). Les composés de l'invention possèdent des propriétés hypnotiques, anxiolytiques et anticonvulsivantes et, par conséquent, peuvent être utilisés pour le traitement d'affections liées aux désordres de la transmission GABAergique, tels que l'anxiété, les troubles du sommeil, l'épilepsie, la spasticité, les contractures musculaires, les troubles cognitifs, les troubles du sevrage vis-à-vis de l'alcoolisme, etc.

EP 0 682 025 A1

Jouve, 18, rue Saint-Denis, 75001 PARIS

La présente invention a pour objet des dérivés de 5,6-di-hydro-4*H*-imidazo[2',1':2,3]imidazo[4,5,1-*ij*]quinoléine et de 4,5-dihydroimidazo[1,2-*a*]pyrrolo[1,2,3-*cd*]benzimidazole, leur préparation et leur application en thérapeutique.

Les composés de l'invention répondent à la formule générale (I)

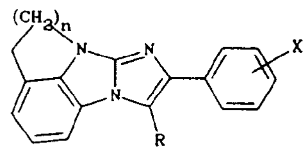

(I)

dans laquelle

n représente le nombre 1 ou 2,

X représente un atome d'hydrogène ou un ou deux atomes ou groupes choisis parmi le fluor, le chlore et les groupes alkyles en $C_1$-$C_3$, alcoxy en $C_1$-$C_3$ et hydroxy, et

R représente soit un atome d'hydrogène, soit un groupe de formule générale $-CH_2-CO_2-R_1$ (dans laquelle $R_1$ représente un atome d'hydrogène ou un groupe alkyle en $C_1$-$C_6$), soit un groupe de formule générale $-CH_2-CO-NR_2R_3$ (dans laquelle $R_2$ et $R_3$, indépendamment l'un de l'autre, représentent chacun un atome d'hydrogène ou un groupe alkyle en $C_1$-$C_3$).

Les composés de l'invention peuvent se présenter à l'état de bases ou de sels.

Parmi les composés de l'invention on préfère ceux dans la formule générale (I) desquels

n représente le nombre 1,

X représente un atome d'halogène ou un groupe méthyle ou méthoxy, et

R représente un groupe de formule générale $-CH_2-CO-NH-CH_3$ ou $-CH_2-CO-N(CH_3)_2$.

Un composé particulièrement intéressant est le 8-(4-fluorophényl)-*N*-méthyl-4,5-dihydroimidazo[1,2-*a*]pyrrolo[1,2,3-*cd*]benzimidazole-9-acétamide.

Conformément à l'invention, les composés de formule générale (I) peuvent être préparés selon un procédé illustré par le schéma qui suit.

On fait réagir un dérivé de formule générale (II), dans laquelle n est tel que défini ci-dessus, avec une 2-halo-1-phényléthanone de formule générale (III), dans laquelle X est tel que défini ci-dessus et Hal représente un atome de chlore ou de brome, pour obtenir un sel de guanidinium de formule générale (IV), que l'on cyclise par chauffage, à une température de 80 à 150°C, dans un solvant tel que, par exemple, l'acide polyphosphorique, pour obtenir un dérivé de formule générale (Ia), laquelle correspond à la formule générale (I) lorsque R représente H.

Si on le désire, on fait ensuite réagir ce dérivé de formule générale (Ia) avec le *N,N*-diméthylglyoxamide (obtenu *in situ* par hydrolyse du 2,2-diéthoxy-*N,N*-diméthylacétamide en présence d'un acide fort, comme décrit dans la demande de brevet EP-251859), dans un solvant protique, tel que l'acide acétique, à une température de 20 à 80°C, pour obtenir un dérivé d'α-hydroxyacétamide de formule générale (V), qu'on traite ensuite avec un polyhalogénure d'acide sulfurique ou phosphorique, par exemple le chlorure de thionyle ou l'oxychlorure de phosphore, ou tout autre agent équivalent, dans un solvant inerte, par exemple un solvant chloré ou éthéré tel que le dichlorométhane ou le tétrahydrofurane, à une température de 20 à 80°C, pour former le dérivé de α-halogénoacétamide correspondant, puis on fait réagir ce dernier soit avec un agent réducteur tel qu'un hydrure alcalin simple ou complexe, par exemple le borohydrure de sodium ou de potassium, dans un solvant protique, par exemple un alcool aliphatique tel que le méthanol ou l'éthanol, ou dans un solvant inerte miscible à l'eau, par exemple le dioxane ou le tétrahydrofurane, à une température de -40 à 40°C, soit avec un agent réducteur tel qu'un hyposulfite ou un dithionite alcalin, par exemple l'hyposulfite ou le dithionite de sodium, ou encore avec l'hydroxyméthylsulfoxylate de sodium (Rongalite®), dans un solvant inerte, par exemple un solvant chloré tel que le dichlorométhane, éventuellement en

Schéma

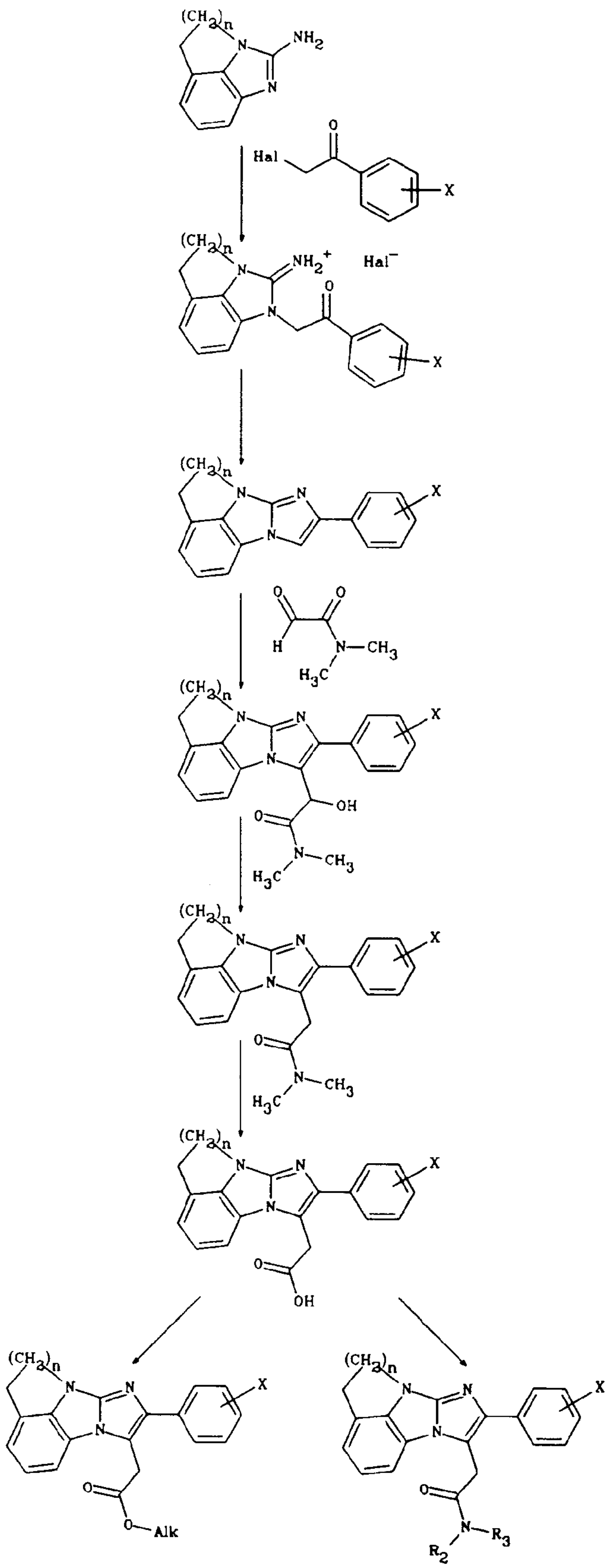

(II)

(III)

(IV)

(Ia)

(V)

(Ib)

(Ic)

(Id)

(Ie)

présence d'un cosolvant inerte miscible à l'eau, par exemple le *N,N*-diméthylformamide ou la *N*-méthylpyrrolidone, à une température de 20 à 40°C, pour obtenir un dérivé de *N,N*-diméthylacétamide de formule générale (Ib), laquelle correspond à la formule générale (I) lorsque R représente $-CH_2-CO-N(CH_3)_2$.

Si on le désire, on transforme le composé de formule générale (Ib) en acide de formule générale (Ic), par hydrolyse au moyen d'une base forte, par exemple la soude ou la potasse, dans un solvant protique, par exemple l'éthanol ou le 2-méthoxyéthanol, en présence d'eau.

Si on le désire, on fait finalement réagir l'acide de formule générale (Ic)

- soit avec le chlorure de thionyle, dans un solvant de type alcool aliphatique en $C_1$-$C_6$, à une température de 20 à 120°C, pour obtenir un ester de formule générale (Id), dans laquelle Alk représente un groupe alkyle en $C_1$-$C_6$,
- soit avec le *N,N'*-carbonyldiimidazole, dans un solvant inerte, par exemple un solvant chloré ou éthéré tel que le dichlorométhane ou le tétrahydrofurane, à une température de 20 à 50°C, pour obtenir l'imidazolide correspondant et on traite ce dernier avec une amine de formule générale $HNR_2R_3$, pour obtenir un amide de formule générale (Ie), dans laquelle $R_2$ et $R_3$ sont tels que définis ci-dessus, à une température de 0 à 25°C.

Le composé de départ de formule générale (II) dans laquelle n représente 1 (c'est-à-dire la 4,5-dihydropyrrolo[1,2,3-*cd*]benzimidazol-2-amine) est nouveau et fait partie de l'invention ; les tentatives de synthèse de ce composé, décrites dans la littérature, par exemple dans *J. Org. Chem.* (1965) **30** 2589, ont été infructueuses, et il n'a jamais été isolé.
Conformément à l'invention il peut être préparé par action d'acide bromohydrocyanique sur la 2,3-dihydro-1*H*-indol-7-amine, à une température de 50 à 70°C, dans un solvant protique, par exemple dans l'eau.

Le composé de départ de formule générale (II) dans laquelle n représente 2 (c'est-à-dire la 5,6-dihydro-4*H*-imidazo[4,5,1-*ij*]quinoléin-2-amine) est connu et décrit sous forme de tautomère imine dans *J. Org. Chem.* (1963) **28** 2581. Il peut être obtenu à partir de 1,2,3,4-tétrahydroquinoléin-8-amine.

La 1,2,3,4-tétrahydroquinoléin-8-amine et la 2,3-dihydro-1*H*-indol-7-amine sont connues et décrites, par exemple, dans *Bull. Soc. Chim. Jpn.* (1989) **62** 2968, *Heterocycles* (1992) **34**(5) 907, *J. Org. Chem.* (1965) **30** 2589, *J. Pr. Soc. N. S. Wales* (1938) **71** 462-474.

Les composés de formule générale (III) sont soit disponibles dans le commerce, soit sont décrits dans la littérature, et peuvent être préparés selon toutes méthodes connues à partir des acétophénones correspondantes et d'agents halogénants adéquats.

Les exemples qui vont suivre illustrent en détail la préparation de quelques composés selon l'invention. Les microanalyses élémentaires, et les spectres I.R. et R.M.N. confirment les structures des composés obtenus.
Les numéros indiqués entre parenthèses dans les titres des exemples correspondent à ceux de la lère colonne du tableau donné plus loin.

Exemple 1 (Composé N°1)

8-Phényl-4,5-dihydroimidazo[1,2-*a*]pyrrolo[1,2,3-*cd*]benzimidazole.

1.1. 1-Acétyl-2,3-dihydro-1*H*-indol-7-amine.

A une solution de 37 g (0,13 mole) de 1-acétyl-5-bromo-7-nitro-2,3-dihydro-1*H*-indole, on ajoute 15 g de palladium sur charbon à 10%, et on soumet la suspension à une hydrogénation dans un appareil de Parr, à une pression de 0,3 MPa et à température ambiante pendant 30 min.

On élimine le catalyseur par filtration et on concentre le filtrat sous pression réduite. On dissout le résidu dans 500 ml d'eau, et on le traite avec un excès de carbonate de sodium jusqu'à pH basique. On collecte l'insoluble par filtration, on le lave à l'eau et on le sèche. On obtient 20,3 g de produit.
Point de fusion : 159°C

1.2. 2,3-Dihydro-1*H*-indol-7-amine.

On chauffe au reflux une solution de 20 g (0,113 mole) de 1-acétyl-2,3-dihydro-1*H*-indol-7-amine dans 80 ml d'acide chlorhydrique 1N pendant 1h.
On la refroidit à température ambiante et on la traite par un excès d'ammoniaque jusqu'à un pH d'environ 8, puis on la traite par 3 fois 100 ml d'éther. On réunit les phases organiques, on les sèche sur sulfate de magnésium, on les filtre et on évapore le solvant sous pression réduite. On distille le résidu huileux, point d'ébullition : 150-160°C sous 133 Pa (1 mm Hg). On obtient 13 g de produit huileux.

1.3. 4,5-Dihydropyrrolo[1,2,3-*cd*]benzimidazol-2-amine.

A une solution de 10,5 g (0,078 mole) de 2,3-dihydro-1*H*-indol-7-amine dans 200 ml d'eau, on ajoute, par petites fractions, 10 g (0,094 mole) d'acide bromohydrocyanique en maintenant la température du milieu réactionel à 60°C. Après refroidissement à température ambiante, on ajoute du carbonate de sodium jusqu'à saturation. On collecte l'insoluble par filtration, on le sèche et on le purifie par chromatographie sur colonne de gel de silice en éluant avec du méthanol. On recristallise la fraction purifiée dans du toluène. On obtient 3,35 g de produit.
Point de fusion : 225-226°C

1.4 Bromure de *N*-[1-(2-oxo-2-phényléthyl)-4,5-dihydropyrrolo[1,2,3-*cd*]benzimidazol-2(1*H*)-ylidène]méthanaminium.

On agite une solution de 3,2 g (0,02 mole) de 4,5-dihydropyrrolo[1,2,3-*cd*]benzimidazol-2-amine et de 4 g (0,02 mole) de 2-bromo-1-phényléthanone dans 350 ml d'éthanol absolu pendant 4h à la température de 80°C, puis 12h à température ambiante. On collecte l'insoluble par filtration et on le sèche. On obtient 6,5 g de sel que l'on utilise tel quel à l'étape suivante.
Point de fusion > 270°C.

1.5. 8-Phényl-4,5-dihydroimidazo[1,2-*a*]pyrrolo[1,2,3-*cd*]benzimidazole.

On chauffe pendant 3h à 120°C, en agitant, un mélange de 4,4 g (0,0123 mole) de bromure de *N*-[1-(2-oxo-2-phényléthyl)-4,5-dihydropyrrolo[1,2,3-*cd*]benzimidazol-2(1*H*)-yl-idène]méthanaminium et de 50 g d'acide polyphosphorique à 84%. On traite ensuite, à température ambiante, le milieu par un mélange d'eau et de glace (500 ml et 500 g) puis on le neutralise au moyen de soude à 30%. On collecte l'insoluble par filtration, on le lave à l'eau et on le sèche à 60°C. On obtient 3 g de produit blanc, que l'on purifie par chromatographie sur colonne de gel de silice en éluant avec un mélange de dichlorométhane/acétone 97/3. On évapore la fraction purifiée sous pression réduite, on recristallise le résidu dans le toluène, on le lave au pentane et on le sèche. On obtient 2,3 g de produit blanc.
Point de fusion : 192-193°C.

<u>Exemple 2</u> (Composé N°2)

*N,N*-Diméthyl-8-phényl-4,5-dihydroimidazo[1,2-*a*]pyrrolo[1,2,3-*cd*]benzimidazole-9-acétamide.

2.1. α-Hydroxy-*N,N*-diméthyl-8-phényl-4,5-dihydroimidazo[1,2-*a*]pyrrolo[1,2,3-*cd*]benzimidazole-9-acétamide.

On chauffe pendant 1h30, sous atmosphère d'azote, à la température de 50°C, 5,26 g (0,03 mole) de 2,2-diéthoxy-*N,N*-diméthylacétamide et 0,7 ml d'acide chlorhydrique concentré dans 50 ml d'acide acétique. On y ajoute 2,5 g (0,03 mole) d'acétate de sodium et on chauffe encore 30 min à 50°C. On ajoute à ce mélange 2,7 g (0,0104 mole) de 8-phényl-4,5-dihydroimidazo[1,2-*a*]pyrrolo[1,2,3-*cd*]benzimidazole, on maintient le chauffage à 50°C pendant 2h, et on laisse au repos pendant 18h.

On évapore le mélange à sec à une température inférieure à 50°C, et on traite le résidu par 200 ml d'eau, 200 ml de dichlorométhane, et un excès de carbonate de sodium jusqu'à neutralité du mélange biphasique. On sépare la phase organique par décantation, on la sèche sur sulfate de magnésium et on évapore le solvant sous pression réduite. On purifie le résidu par chromatographie sur colonne de gel de silice en éluant avec un mélange de chloroforme/acétone 95/5. On obtient un produit huileux qui cristallise par traitement à l'éther diéthylique. On isole, après séchage, 2,7 g de produit blanc.
Point de fusion : 187-189°C.

2.2. *N,N*-Diméthyl-8-phényl-4,5-dihydroimidazo[1,2-*a*]pyrrolo[1,2,3-*cd*]benzimidazole-9-acétamide.

2.2.1. α-Chloro-*N,N*-diméthyl-8-phényl-4,5-dihydroimidazo[1,2-*a*]pyrrolo[1,2,3-*cd*]benzimidazole-9-acétamide.

A 2,6 g (0,0072 mole) de α-hydroxy-*N,N*-diméthyl-8-phényl-4,5-dihydroimidazo[1,2-*a*]pyrrolo[1,2,3-*cd*]benzimidazole-9-acétamide, dissous dans 50 ml de dichlorométhane sec, on ajoute, en agitant, 15 g (0,126

mole) de chlorure de thionyle. On maintient l'agitation pendant 4h, sous atmosphère d'azote, et on laisse une nuit au repos.
On évapore le mélange à sec, sous pression réduite, on reprend le résidu par du toluène et on l'évapore à nouveau. On triture le résidu dans l'éther diéthylique, on le sèche rapidement et on l'utilise tel quel à l'étape suivante.

2.2.2. *N,N*-Diméthyl-8-phény1-4,5-dihydroimidazo[1,2-*a*]-pyrrolo[1,2,3-*cd*]benzimidazole-9-acétamide.

On dissout le α-chloro-*N,N*-diméthyl-8-phényl-4,5-dihydroimidazo[1,2-*a*]pyrrolo[1,2,3-*cd*]benzimidazole-9-acétamide obtenu précédemment dans un mélange de 50 ml de dichlorométhane sec et de 20 ml de *N,N*-diméthylformamide. On ajoute à cette solution 3,08 g (0,002 mole) de Rongalite® et on agite pendant 15h à température ambiante.
On évapore le solvant à sec, à une température de l'ordre de 50°C, et on reprend le résidu par une solution saturée d'hydrogénocarbonate de sodium. On traite la phase aqueuse par du dichlorométhane, on sépare la phase organique, on la sèche sur sulfate de magnésium et on évapore le solvant sous pression réduite. On purifie le résidu par chromatographie sur colonne de gel de silice en éluant avec un mélange de dichlorométhane/acétone 95/5, on recristallise la fraction purifiée dans l'alcool isopropylique et on le sèche à 100°C sous pression réduite.
On obtient 0,85 g de solide.
Point de fusion : 224-225°C

Exemple 3 (Composé N°3)

Acide 8-phényl-4,5-dihydroimidazo[1,2-*a*]pyrrolo[1,2,3-*cd*]benzimidazole-9-acétique.
On chauffe pendant 6h au reflux un mélange de 0,45 g (0,0013 mole) de *N,N*-diméthyl-8-phényl-4,5-dihydroimidazo[1,2-*a*]pyrrolo[1,2,3-*cd*]benzimidazole-9-acétamide et 0,28 g d'hydroxyde de sodium (0,007 mole) dissous dans un mélange de 10 ml de 2-méthoxyéthanol et 2 ml d'eau.
On évapore les solvants sous pression réduite et on reprend le résidu par 50 ml d'eau. On élimine un insoluble par filtration, on acidifie le filtrat au moyen d'acide acétique. On collecte l'insoluble par filtration, on le lave à l'eau jusqu'à pH 5 et on le sèche à 80°C sous pression réduite pendant 8h.
On obtient 0,4 g de solide blanc-beige.
Point de fusion : 270-275°C (avec décomposition).

Exemple 4 (Composé N°4)

8-Phényl-4,5-dihydroimidazo[1,2,3-*cd*]benzimidazole-9-acétate de méthyle.
A une suspension de 0,02 g d'acide 8-phényl-4,5-dihydroimidazo[1,2-*a*]pyrrolo[1,2,3-*cd*]benzimidazole-9-acétique dans 10 ml de méthanol, refroidie à 0°C, on ajoute 0,2 ml de chlorure de thionyle, et on laisse le mélange sous agitation à température ambiante pendant 24h.
On évapore le solvant sous pression réduite, on reprend le résidu avec un excès de solution aqueuse saturée d'hydrogénocarbonate de sodium jusqu'à pH>7, et on l'extrait au dichlorométhane. On sépare la phase organique, on la sèche sur sulfate de magnésium, on la filtre et on évapore le solvant sous pression réduite.
On reprend le résidu avec de l'éther diisopropylique, on le laisse cristalliser au froid et on obtient 0,015 g de produit.
Point de fusion : 164-165°C.

Exemple 5 (Composé N°5)

*N*-Méthyl-8-phényl-4,5-dihydroimidazo[1,2-*a*]pyrrolo[1,2,3-*cd*]benzimidazole-9-acétamide.
On chauffe pendant 4h à une température de 50°C, sous atmosphère d'azote, 0,37 g (0,00116 mole) d'acide 8-phényl-4,5-dihydroimidazo[1,2-*a*]pyrrolo[1,2,3-*cd*]benzimidazole-9-acétique et 0,23 g (0,0014 mole) de *N,N'*-carbonyldiimidazole dans 15 ml de tétrahydrofurane sec.
Ensuite, à froid, on fait passer, durant 30 min, un courant de méthylamine gazeuse sèche dans le milieu réactionnel, on agite pendant 4h à température ambiante et on laisse au repos pendant une nuit.
On évapore le solvant et l'excès d'amine sous pression réduite, on reprend le résidu par 30 ml d'eau et 100 ml de dichlorométhane et on l'agite pendant quelques minutes. On sépare la phase organique, on la sèche sur sulfate de magnésium et on évapore le solvant sous pression réduite. On recristallise le résidu dans l'éthanol absolu, on lave les cristaux obtenus à l'éther et on les sèche.

On obtient 0,26 g de solide.
Point de fusion : 269-270°C

Exemple 6 (Composé N°6)

9-Phényl-5,6-dihydro-4*H*-imidazo[2',1':2,3]imidazo[4,5,1-*ij*]quinoléine et son chlorhydrate.

6.1 1,2,3,4-Tétrahydroquinoléin- 8-amine.

A une solution, maintenue à une température de 50 à 60°C, de 45 g (0,312 mole) de quinoléin-8-amine dans 1 l d'éthanol absolu, on ajoute par petites fractions, 80 g (3,5 mole) de sodium. En fin d'addition on chauffe le mélange jusqu'à disparition du sodium, puis on le refroidit par un mélange d'eau et de glace. On traite ensuite le mélange réactionnel par 200 ml d'eau, et on évapore le tout sous pression réduite. On traite le résidu solide avec trois fois 300 ml d'éther diéthylique, on réunit les phases organiques, on les sèche sur sulfate de magnésium et on évapore le solvant. On purifie le résidu par chromatographie sur colonne de gel de silice en éluant avec un mélange de dichlorométhane/éther diéthylique 90/10 à 85/15. On évapore la fraction purifiée sous pression réduite et on sèche le résidu à 60°C sous vide. On obtient 30 g d'huile jaune que l'on utilise telle quelle à l'étape suivante.

6.2. 5,6-Dihydro-4*H*-imidazo[4,5,1-*ij*]quinoléin-2-amine.

A une solution de 14,8 g (0,1 mole) de 1,2,3,4-tétrahydroquinoléin-8-amine dans 100 ml de méthanol, refroidie à 0°C, on ajoute une solution de 11,1 g (0,105 mole) d'acide bromohydrocyanique dans 50 ml de méthanol. La réaction est exothermique. Après 1h d'agitation à température ambiante et une nuit au repos, on ajoute au mélange 100 ml d'éther diéthylique et on l'agite pendant 30 min.
On collecte le bromhydrate du produit attendu par filtration, on le lave à l'éther diéthylique et on le sèche grossièrement. On le dissout dans 200 ml d'eau et on ajoute en agitant le mélange vigoureusement, 25 ml de soude à 30%. Après 1h d'agitation, on filtre la suspension obtenue et on lave le précipité à l'eau et on le sèche.
On obtient 13,6 g de solide.
Point de fusion : 200°C (littérature : 201-202°C).

6.3. Bromure de *N*-[1-(2-oxo-2-phényléthyl)-1,4,5,6-tétrahydro-2*H*-imidazo[4,5,1-*ij*]quinoléin-2-ylidène]méthanaminium.

On chauffe pendant 4h, sous atmosphère d'azote, au reflux du solvant, 13,5 g (0,0779 mole) de 5,6-dihydro-4*H*-imidazo[4,5,1-*ij*]quinoléin-2-amine, 15,9 g (0,08 mole) de 2-bromo-1-phényléthanone dans 1l d'éther absolu.
Après une nuit au repos on collecte l'insoluble par filtration, on le lave à l'éthanol puis à l'éther diéthylique et on le sèche.
On obtient 21,5 g du sel attendu.
Point de fusion >270°C

6.4 9-Phényl-5,6-dihydro-4*H*-imidazo[2',1':2,3]imidazo[4,5,1-*ij*]quinoléine, et son chlorhydrate

On chauffe pendant 3h à 120°C, en agitant, un mélange de 21 g (0,0564 mole) de bromure de *N*-[1-(2-oxo-2-phényléthyl)-1,4,5,6-tétrahydro-2*H*-imidazo[4,5,1-*ij*]quinoléin-2-ylidène]méthanaminium dans 250 g d'acide polyphosphorique à 84%. On verse le mélange encore chaud (80-100°C) dans un mélange de 500 ml d'eau et 500 g de glace maintenu sous agitation. On neutralise la solution, à froid, au moyen de soude à 30%. On filtre la suspension qui en résulte, on lave l'insoluble trois fois à l'eau, et on le sèche sous pression réduite.
On obtient 15 g de produit que l'on purifie par chromatographie sur colonne de gel de silice en éluant avec du dichlorométhane.
On obtient 11,5 g de solide.
Point de fusion : 103-104°C.

Si on le désire, on peut préparer le chlorhydrate à partir de 1 g (0,00366 mole) de base dissoute dans 25 ml d'éthanol absolu et 3 ml d'une solution 1,5 N d'acide chlorhydrique gazeux dans l'éthanol sec. On recristallise le chlorhydrate dans un mélange de 2-méthoxyéthanol/eau 90/10, on lave les cristaux obtenus par de l'alcool éthylique et de l'éther diéthylique, et on les sèche pendant 10h à 100°C sous pression réduite.
On obtient 0,8 g de produit blanc.

Point de fusion : 295-300°C (avec décomposition)

Exemple 7 (Composé N°7)

*N,N*-Diméthyl-9-phényl-5,6-dihydro-4*H*-imidazo[2',1':2,3]imidazo[4,5,1-*ij*]quinoléine-10-acétamide.

7.1. α-Hydroxy-*N,N*-diméthyl-9-phényl-5,6-dihydro-4*H*-imidazo[2',1':2,3]imidazo[4,5,1-*ij*]quinoléine-10-acétamide et son chlorhydrate.

On chauffe pendant 2h à 50°C, sous atmosphère d'azote, un mélange de 17,5 g (0,1 mole) de 2,2-diéthoxy-*N,N*-diméthylacétamide, de 2 ml d'acide chlorhydrique concentré (35%) et 170 ml d'acide acétique glacial. On ajoute ensuite 8,2 g d'acétate de sodium anhydre et on agite le mélange pendant 30 min à 50°C. Finalement, on ajoute à ce mélange, refroidi à 0°C, 9 g (0,0329 mole) de 9-phényl-5,6-dihydro-4*H*-imidazo[2',1':2,3]imidazo[4,5,1-*ij*]quinoléine, on l'agite pendant 2h à température ambiante et on le laisse une nuit au repos. On évapore l'acide acétique sous pression réduite, on reprend le résidu par 100 ml d'eau et 200 ml de dichlorométhane. On traite ce mélange biphasique, maintenu sous agitation vigoureuse, par du carbonate de sodium, par petites portions jusqu'à pH alcalin. On sépare la phase organique, on la sèche sur sulfate de sodium et on évapore le solvant sous pression réduite. On purifie le résidu par chromatographie sur colonne de gel de silice en éluant avec un mélange de dichlorométhane/acétone 95/5. On évapore la fraction purifiée sous pression réduite, et on obtient 9,2 g d'huile que l'on utilise telle quelle à l'étape suivante. Si on le désire on peut en préparer le chlorhydrate à partir de 0,1 g de base et un équivalent d'acide chlorhydrique gazeux dissous dans l'éther diéthylique. On recristallise le chlorhydrate dans l'acétonitrile et on obtient 0,075 g de solide blanc.
Point de fusion : 198-200°C

7.2. *N,N*-Diméthyl-9-phény1-5,6-dihydro-4*H*-imidazo[2',1':2,3]imidazo[4,5,1-*ij*]quinoléine-10-acétamide.

a) A une solution de 9 g (0,024 mole) de α-hydroxy-*N,N*-diméthyl-9-phényl-5,6-dihydro-4*H*-imidazo[2',1':2,3]imidazo[4,5,1-*ij*]quinoléine-10-acétamide dans 200 ml de dichlorométhane, on ajoute 60 g, soit 36,5 ml, de chlorure de thionyle. Après 4h d'agitation à température ambiante et une nuit au repos, on évapore le solvant et l'excès de chlorure de thionyle sous pression réduite. On reprend le résidu par du toluène et on évapore à nouveau. On obtient le chlorhydrate à l'état de produit gommeux et on l'utilise tel quel à l'étape suivante.
b) On dissout le produit gommeux obtenu dans l'étape précédente dans un mélange de 200 ml de dichlorométhane et 150 ml de *N,N*-diméthylformamide secs, on y ajoute 11,1 g (0,072 mole) de Rongalite® et on agite le mélange pendant 8h à température ambiante.
On évapore les solvants sous pression réduite, sans dépasser la température de 50°C, on reprend le résidu par 200 ml de solution aqueuse saturée d'hydrogénocarbonate de sodium, et on le traite par 200 ml de dichlorométhane. On sépare la phase organique, on la sèche sur sulfate de sodium, on évapore le solvant sous pression réduite, et on purifie le résidu par chromatographie sur colonne de gel de silice en éluant avec un mélange de dichlorométhane/méthanol 97/3. On concentre la fraction purifiée et on recristallise le résidu dans l'acétate d'éthyle. On lave les cristaux à l'éther diéthylique, on les sèche pendant 8h à 100°C sous pression réduite, et on obtient 6,4 g de solide blanc.
Point de fusion : 216-217°C

Exemple 8 (Composé N°8)

Acide 9-phényl-5,6-dihydro-4*H*-imidazo[2',1':2,3]imidazo[4,5,1-*ij*]quinoléine-10-acétique.

A une solution de 5,5 g (0,0153 mole) de *N,N*-diméthyl-9-phényl-5,6-dihydro-4*H*-imidazo[2',1':2,3]imidazo[4,5,1-*ij*]quinoléine-10-acétamide dans 150 ml de 2-méthoxyéthanol, on ajoute une solution de 6 g de soude dans 25 ml d'eau et on porte le mélange au reflux du solvant pendant 8h.
On refroidit le mélange, on évapore le solvant sous pression réduite à 60°C, on reprend le résidu par 200 ml d'eau et on le traite par de l'acide chlorhydrique 6N jusqu'à un pH de 8,5 à 9. On élimine l'insoluble que se forme par filtration, on amène le filtrat à un pH de 3,8 à 4 par de l'acide chlorhydrique dilué, on collecte l'insoluble par filtration, on le lave à l'eau et on le sèche à 60-80°C sous pression réduite.
On obtient 4,7 g de solide.
Point de fusion : 246-248°C (avec décomposition)

Exemple 9 (Composé N°9)

9-Phényl-5,6-dihydro-4*H*-imidazo[2',1':3,2]imidazo[4,5,1-*ij*]quinoléine-10-acétamide.

On chauffe pendant 1h à 50°C, en agitant, un mélange de 1,1 g (0,0033 mole) d'acide 9-phényl-5,6-dihydro-4*H*-imidazo[2',1':2,3]imidazo[4,5,1-*ij*]quinoléine-10-acétique et 0,58 g (0,0036 mole) de *N,N'*-carbonyldiimidazole dans 50 ml de tétrahydrofurane sec. On refroidit le mélange, on le traite par un courant d'ammoniac pendant 30 min, on l'agite pendant 2h et on le laisse une nuit au repos.

On évapore le solvant sous pression réduite, on lave le résidu par de l'eau, puis avec une solution aqueuse saturée d'hydrogénocarbonate de sodium, puis encore avec de l'eau, et finalement on le sèche à 100°C sous pression réduite. On le recristallise dans l'éthanol absolu et on obtient 0,85 g de solide.

Point de fusion : 249-250°C

Exemple 10 (Composé N°10)

*N*-Méthyl-9-phényl-5,6-dihydro-4*H*-imidazo[2',1':2,3]imidazo[4,5,1-*ij*]quinoléine-10-acétamide.

On procède de la manière décrite dans l'exemple 5 en partant de 1,1 g (0,0033 mole) d'acide 9-phényl-5,6-dihydro-4*H*-imidazo[2',1':2,3]imidazo[4,5,1-*ij*]quinoléine-10-acétique avec un excès de méthylamine gazeuse. On obtient après cristallisation dans l'éthanol absolu 0,73 g de solide.

Point de fusion : 247-248°C

Exemple 11 (Composé N°11)

9-Phényl-5,6-dihydro-4*H*-imidazo[2',1':2,3]imidazo[4,5,1-*ij*]quinoléine-10-acétate de méthyle.

A une suspension de 0,5 g (0,0015 mole) d'acide 9-phényl-5,6-dihydro-4*H*-imidazo[2',1':2,3]imidazo[4,5,1-*ij*]quinoléine-10-acétique dans 20 ml de méthanol, refroidie à 0°C, on ajoute, goutte à goutte, 0,5 ml de chlorure de thionyle, et on chauffe le mélange à 60°C et sous atmosphère d'azote sec pendant 8h.

On évapore le solvant sous pression réduite, on reprend le résidu avec un excès de solution aqueuse saturée d'hydrogénocarbonate de sodium jusqu'à pH>7, et on l'extrait à l'éther diéthylique. On sépare la phase organique, on la sèche sur sulfate de magnésium, on la filtre et on évapore le solvant sous pression réduite.

On reprend le résidu huileux avec de l'éther diisopropylique, on le laisse cristalliser au froid et on obtient 0,38 g de solide.

Point de fusion : 123-124°C.

Exemple 12 (Composé N°16)

8-(4-Fluorophényl)-4,5-dihydroimidazo[1,2-*a*]pyrrolo[1,2,3-*cd*]benzimidazole.

A une solution de 28 g (0,2 mole) de 1-(4-fluorophényl)-éthanone dans 200 ml de chloroforme on ajoute, goutte à goutte, 33,6 g, soit 10,8 ml (0,21 mole) de brome. Après 15 min à température ambiante on ajoute 100 ml d'eau, on sépare la phase organique, on la lave avec 50 ml d'eau, on la sèche sur sulfate de magnésium, on la filtre, on évapore le solvant sous pression réduite, on dissout le résidu dans 200 ml de pentane à 40°C, on refroidit la solution à -5°C en l'agitant, on filtre les cristaux et on les sèche sous pression réduite.

On obtient 32,1 g de produit.

Point de fusion : 47-48°C.

12.2. Bromure de *N*-[1-[2-(4-fluorophényl)-2-oxoéthyl]-4,5-dihydropyrrolo[1,2,3-*cd*]benzimidazol-2(1*H*)-ylidène]méthanaminium.

A une solution de 9,5 g (0,06 mole) de 4,5-dihydropyrrolo[1,2,3-*cd*]benzimidazol-2-amine dans 450 ml d'éthanol on ajoute une solution de 13 g (0,06 mole) de 2-bromo-1-(4-fluorophényl)éthanone dans 50 ml d'éthanol, on maintient l'agitation pendant 6h, on ajoute 500 ml d'éther diéthylique et on laisse reposer pendant une nuit.

On sépare le solide par filtration, on le lave à l'éther diéthylique et on le sèche.

On obtient 20,7 g de produit.

Point de fusion : 260-265°C (décomposition)

12.3. 8-(4-Fluorophényl)-4,5-dihydroimidazo[1,2-*a*]pyrrolo[1,2,3-*cd*]benzimidazole.

On chauffe pendant 4h à 120°C, en agitant, un mélange de 20,5 g (0,0545 mole) de bromure de *N*-[1-(2-(4-fluorophényl)-2-oxoéthyl)-4,5-dihydropyrrolo[1,2,3-*cd*]benzimidazol-2(1*H*)-ylidène]méthanaminium et de 200 g d'acide polyphosphorique à 84%. On traite ensuite, à température ambiante, le milieu par un mélange d'eau et de glace (500 ml et 500 g) puis on le neutralise au moyen de soude à 30% (pH>10). On collecte l'insoluble par filtration, on le lave à l'eau et on le sèche à 60°C.
On obtient 14,4 g de produit.
Point de fusion : 189-190°C.

Exemple 13 (Composé N°17)

*N,N*-Diméthyl-8-(4-fluorophényl)-4,5-dihydroimidazo[1,2-*a*]pyrrolo[1,2,3-*cd*]benzimidazole-9-acétamide.

13.1. 8-(4-Fluorophényl)-α-hydroxy-*N,N*-diméthyl-4,5-dihydroimidazo[1,2-*a*]pyrrolo[1,2,3-*cd*]benzimidazole-9-acétamide.

On chauffe pendant 2h, sous atmosphère d'azote, à la température de 40°C, 27,2 g (0,155 mole) de 2,2-diéthoxy-*N,N*-diméthylacétamide et 3 ml d'acide chlorhydrique concentré dans 250 ml d'acide acétique. On y ajoute 12,7 g (0,155 mole) d'acétate de sodium et on chauffe encore 15 min à 40°C.
On ajoute à ce mélange 14,3 g (0,0516 mole) de 8-(4-fluorophényl)-4,5-dihydroimidazo[1,2-*a*]pyrrolo[1,2,3-*cd*]benzimidazole, on maintient le chauffage à 40°C pendant 4h, et on laisse au repos pendant une nuit.
On évapore l'acide acétique sous pression réduite à une température inférieure à 45°C, on traite le résidu par 250 ml de dichlorométhane, et on ajoute, goutte à goutte, une solution aqueuse à 10% de carbonate de sodium jusqu'à pH alcalin.
On sépare la phase organique par décantation, on la sèche sur sulfate de magnésium, on évapore le solvant sous pression réduite, et on triture le résidu cristallisé dans un mélange de pentane/éther diéthylique 50/50.
On obtient 17,5 g de produit.
Point de fusion : 197-198°C.

13.2. *N,N*-Diméthyl-8-(4-fluorophényl)-4,5-dihydroimidazo[1,2-*a*]pyrrolo[1,2,3-*cd*]benzimidazole-9-acétamide.

13.2.1. α-Chloro-*N,N*-diméthy1-8-(4-fluorophényl)-4,5-dihydroimidazo[1,2-*a*]pyrrolo[1,2,3-*cd*]benzimidazole-9-acétamide.

A une solution de 17,3 g (0,0457 mole) de 8-(4-fluorophényl)-α-hydroxy-*N,N*-diméthyl-4,5-dihydroimidazo[1,2-*a*]pyrrolo[1,2,3-*cd*]benzimidazole-9-acétamide dans 500 ml de dichlorométhane on ajoute, goutte à goutte, 50 ml (-0,7 mole) de chlorure de thionyle et on agite le mélange pendant 8h à température ambiante.
On évapore le mélange sous pression réduite, on reprend le résidu avec du toluène et on évapore ce dernier.
On triture le résidu dans l'éther diéthylique, on l'essore, on le lave à l'éther diéthylique et on le sèche.
On obtient 19,2 g de produit.
Point de fusion : 180-185°C (décomposition).

13.2.2. *N,N*-Diméthyl-8-(4-fluorophényl)-4,5-dihydroimidazo[1,2-*a*]pyrrolo[1,2,3-*cd*]benzimidazole-9-acétamide.

A une solution de 19 g (0,0438 mole) de α-chloro-*N,N*-diméthyl-8-(4-fluorophényl)-4,5-dihydroimidazo[1,2-*a*]pyrrolo[1,2,3-*cd*]benzimidazole-9-acétamide dans 500 ml de dichlorométhane, agitée sous atmosphère d'azote, on ajoute 27 g (0,175 mole) de Rongalite®, et on agite le mélange à température ambiante pendant 20h.
On ajoute 200 ml de solution aqueuse saturée d'hydrogénocarbonate de sodium, on sépare la phase organique, on la lave à l'eau, on la sèche sur sulfate de magnésium, on la filtre, on évapore le solvant sous pression réduite et on purifie le résidu par chromatographie sur colonne de gel de silice en éluant avec un mélange de dichlorométhane/méthanol 95/5. Après recristallisation dans le 2-méthoxyéthanol, lavage à l'éthanol, lavage à l'éther diéthylique et séchage on obtient 10,6 g de produit.
Point de fusion : 259-260°C.

Exemple 14 (Composé N°18)

Acide 8-(4-fluorophényl)-4,5-dihydroimidazo[1,2-*a*]pyrrolo[1,2,3-*cd*]benzimidazole-9-acétique.

Sous atmosphère d'azote on chauffe un mélange de 4 g (0,011 mole) de *N,N*-diméthyl-8-(4-fluorophényl)-4,5-dihydroimidazo[1,2-*a*]pyrrolo[1,2,3-*cd*]benzimidazole-9-acétamide, 2,2 g (~0,055 mole) de soude, 15 ml d'eau et 100 ml de 2-méthoxyéthanol pendant 8h à la température du reflux.

On évapore les solvants, on reprend le résidu avec 250 ml d'eau, un élimine un insoluble par filtration, on ajoute au filtrat de l'acide acétique jusqu'à pH=5, on recueille le solide par filtration, on le lave trois fois à l'eau et on le sèche.

On obtient 3,5 g de produit.

Point de fusion : 225-227°C.

Exemple 15 (Composé N°19)

8-(4-Fluorophényl)-*N*-méthyl-4,5-dihydroimidazo[1,2-*a*]pyrrolo[1,2,3-*cd*]benzimidazole-9-acétamide.

Sous atmosphère d'azote on chauffe un mélange de 3,3 g (0,00984 mole) d'acide 8-(4-fluorophényl)-4,5-dihydroimidazo[1,2-*a*]pyrrolo[1,2,3-*cd*]benzimidazole-9-acétique, 1,95 g (0,012 mole) de *N,N'*-carbonyldiimidazole et 200 ml de tétrahydrofurane sec à 50°C pendant 4h.

Ensuite, à froid, on fait passer dans le mélange un courant de méthylamine gazeuse sèche, durant 30 min, on l'agite pendant 2h et on le laisse reposer pendant une nuit.

On évapore le mélange sous pression réduite, on reprend le résidu avec 100 ml d'eau, on sépare le solide par filtration, on le lave à l'eau, on le sèche et on le purifie par chromatographie sur colonne de gel de silice en éluant avec un mélange de dichlorométhane/méthanol 97/3, puis par recristallisation dans un mélange d'éthanol/2-méthoxyéthanol 80/20.

On obtient finalement 1,6 g de produit.

Point de fusion : 265-266°C.

Le tableau qui suit illustre les structures chimiques et les propriétés physiques de quelques composés selon l'invention.

Tableau

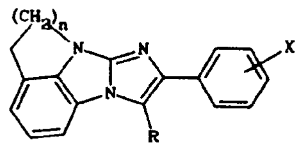

(I)

| N° | n | X | R | Sel | F (°C) |
|---|---|---|---|---|---|
| 1 | 1 | H | H | - | 192-193 |
| 2 | 1 | H | $-CH_2CON(CH_3)_2$ | - | 224-225 |
| 3 | 1 | H | $-CH_2COOH$ | - | 270-275 (d) |
| 4 | 1 | H | $-CH_2COOCH_3$ | - | 164-165 |
| 5 | 1 | H | $-CH_2CONHCH_3$ | - | 269-270 |
| 6 | 2 | H | H | - | 103-104 |
| | | | | HCl | 295-300 (d) |
| 7 | 2 | H | $-CH_2CON(CH_3)_2$ | - | 216-217 |
| 8 | 2 | H | $-CH_2COOH$ | - | 246-248 (d) |
| 9 | 2 | H | $-CH_2CONH_2$ | - | 249-250 |
| 10 | 2 | H | $-CH_2CONHCH_3$ | - | 247-248 |
| 11 | 2 | H | $-CH_2COOCH_3$ | - | 123-124 |
| 12 | 1 | 3-F | H | - | 169-170 |
| 13 | 1 | 3-F | $-CH_2CON(CH_3)_2$ | - | 222-224 |
| 14 | 1 | 3-F | $-CH_2COOH$ | - | 274-277 (d) |
| 15 | 1 | 3-F | $-CH_2CONHCH_3$ | - | 296-297 |
| 16 | 1 | 4-F | H | - | 189-190 |
| 17 | 1 | 4-F | $-CH_2CON(CH_3)_2$ | - | 259-260 |
| 18 | 1 | 4-F | $-CH_2COOH$ | - | 225-227 |
| 19 | 1 | 4-F | $-CH_2CONHCH_3$ | - | 265-266 |
| 20 | 1 | 4-F | $-CH_2CONHCH_2CH_3$ | - | 267-268 (d) |
| 21 | 1 | 4-F | $-CH_2CONHCH_2CH_2CH_3$ | - | 256-257 (d) |

| N° | n | X | R | Sel | F (°C) |
|---|---|---|---|---|---|
| 22 | 1 | 4-Cl | H | - | 209-210 |
| 23 | 1 | 4-Cl | $-CH_2CON(CH_3)_2$ | - | 277-278 |
| 24 | 1 | 4-Cl | $-CH_2COOH$ | - | 253 |
| 25 | 1 | 4-Cl | $-CH_2CONHCH_3$ | - | 289-290 |
| 26 | 1 | $4-CH_3$ | H | - | 205-207 |
| 27 | 1 | $4-CH_3$ | $-CH_2CON(CH_3)_2$ | - | 243-244 |
| 28 | 1 | $4-CH_3$ | $-CH_2COOH$ | - | 280-285 (d) |
| 29 | 1 | $4-CH_3$ | $-CH_2CONHCH_3$ | - | 279-280 |
| 30 | 1 | $4-OCH_3$ | H | - | 209-210 |
| 31 | 1 | $4-OCH_3$ | $-CH_2CON(CH_3)_2$ | - | 216-217 |
| 32 | 1 | $4-OCH_3$ | $-CH_2COOH$ | - | 223-225 (d) |
| 33 | 1 | $4-OCH_3$ | $-CH_2CONHCH_3$ | - | 266-267 (d) |
| 34 | 1 | 4-OH | $-CH_2CON(CH_3)_2$ | - | 259-260 |
| 35 | 1 | 4-OH | $-CH_2CONHCH_3$ | - | 308-310 |
| 36 | 1 | 3-F, $4-OCH_3$ | H | - | 209-211 |
| 37 | 1 | 3-F, $4-OCH_3$ | $-CH_2CON(CH_3)_2$ | - | 235-237 |
| 38 | 1 | 3-F, $4-OCH_3$ | $-CH_2COOH$ | - | > 250 (d) |
| 39 | 1 | 3-F, $4-OCH_3$ | $-CH_2CONHCH_3$ | - | 268-270 (d) |

Légende : dans la colonne "Sel", "-" désigne un composé à l'état de base et "HCl" désigne un chlorhydrate ; dans la colonne "F (°C)", "(d)" désigne un point de fusion avec décomposition.

Les composés de l'invention ont été soumis à des essais pharmacologiques qui ont mis en évidence leur intérêt comme substances à activités thérapeutiques.

Etude des liaisons membranaires vis-à-vis des récepteurs $\omega_1$ (benzodiazépiniques de type 1) et $\omega_2$ (benzodiazépiniques de type 2).

L'affinité des composés pour les récepteurs $\omega_1$ du cervelet et $\omega_2$ de la moëlle épinière a été déterminée

selon une variante de la méthode décrite par S. Z. Langer et S. Arbilla dans *Fund. Clin. Pharmacol.*, **2**, 159-170 (1988), avec utilisation de $^3$H-flumazenil au lieu de $^3$H-diazepam comme radioligand. On homogénéise le tissu du cervelet ou de la moëlle épinière pendant 60 s dans 120 ou 30 volumes, respectivement, de tampon glacé (50 mM Tris/HCl, pH 7,4, 120 mM NaCl, 5 mM KCl) puis, après dilution à 1/3, on fait incuber la suspension avec du $^3$H-flumazenil (activité spécifique 78 Ci/mmole, New England Nuclear) à une concentration de 1 nM et avec les composés de l'invention à différentes concentrations, dans un volume final de 525 µl. Après 30 minutes d'incubation à 0°C on filtre les échantillons sous vide sur des filtres Whatman GF/B® et on les lave immédiatement avec du tampon glacé. La liaison spécifique du $^3$H-flumazenil est déterminée en présence de diazépam 1 µM non marqué. On analyse les données selon les méthodes usuelles et on calcule la concentration $CI_{50}$, concentration qui inhibe de 50% la liaison du $^3$H-flumazenil.
Les $CI_{50}$ des composés de l'invention se situent, dans ces essais, entre 1 et 1000 nM.

Etude de l'activité anticonvulsivante.

Activité vis-à-vis des convulsions cloniques induites chez le rat par injection de pentétrazol.

Le protocole de cet essai est une modification de celui décrit par E. A. Swinyard et J. H. Woodhead dans *Antiepi- leptic Drugs*, Raven Press, New York, 111-126 (1982).

Les produits à tester sont administrés aux animaux par voie intrapéritonéale 30 minutes avant une injection intraveineuse d'une dose de 20 mg/kg de pentétrazol. Immédiatement après l'injection on note pendant 5 minutes le nombre d'animaux présentant des convulsions cloniques.
Les résultats sont exprimés par la $DA_{50}$, dose qui protège 50% des animaux, calculée selon la méthode de J. T. Lichtfield et F. Wilcoxon (*J. Pharm. Exp. Ther.*, **96**, 99-113 (1949)) à partir de 3 ou 4 doses administrées chacune à un groupe de 8 à 10 souris.
Les $DA_{50}$ des composés de l'invention se situent, dans cet essai, entre 0,5 et 10 mg/kg par la voie intrapéritonéale.

Etude de l'activité anticonvulsivante.

Activité vis-à-vis des convulsions induites chez la souris par l'isoniazide.

L'activité intrinsèque des composés est déterminée par le temps de latence d'apparition des convulsions induites par l'administration sous-cutanée d'isoniazide (800 mg/kg) simultanément avec le composé à tester, injecté par voie intrapéritonéale, selon le protocole décrit par G. Perrault, E. Morel, D. Sanger et B. Zivkovic dans *Eur. J. Pharmacol.*, **156**, 189-196 (1988). Les résultats sont exprimés par la $DA_{50}$, dose qui produit 50% de l'effet maximal, par rapport aux animaux témoins, déterminée à partir de 3 ou 4 doses administrées chacune à un groupe de 8 à 10 souris.
Les $DA_{50}$, des composés de l'invention se situent, dans cet essai, entre 1 et 100 mg/kg par la voie intrapéritonéale et, selon les composés, l'effet maximal peut aller jusqu'à 350%.

Etude de l'activité anxiolytique.

L'activité anxiolytique est évaluée chez le rat dans le test de conflit de prise de boisson, selon la méthode décrite par J. R. Vogel, B. Beer et D. E. Clody dans *Psychopharmacologia* (Berl.), **21**, 1-7 (1971).
Après une diète hydrique de 48h, le rat est placé dans une enceinte isolée du bruit et équipée d'une pipette d'eau reliée à un anxiomètre délivrant un léger choc électrique tous les 20 coups de langue. Le nombre de chocs reçus est automatiquement compté pendant 3 minutes, et permet d'évaluer l'activité anxiolytique des composés testés. Les résultats sont exprimés par la dose efficace minimale (DEM), dose qui produit une augmentation significative du nombre de chocs reçus, par rapport au nombre observé chez les animaux témoins.
Les DEM des composés de l'invention se situent, dans cet essai, entre 1 et 50 mg/kg par la voie intrapéritonéale ou orale.

Etude de l'activité hypnotique.

L'activité sédative ou hypnotique des composés a été déterminée par l'observation de leur action sur l'électrocorticogramme du rat, selon la méthode décrite par H. Depoortere, *Rev. E.E.G. Neurophysiol.*, **10**, 3, 207-214 (1980) et par H. Depoortere et M. Decobert, *J. Pharmacol. (Paris)*, **14**, 2, 195-265 (1983).
Les produits à étudier ont été administrés par voie intrapéritonéale à doses croissantes. Ils induisent des tracés

de sommeil à des doses allant de 1 à 30 mg/kg.

Les résultats des essais effectués sur les composés de l'invention montrent que, in vitro, ils déplacent le $^3H$-flumazénil de ses sites de liaison spécifiques au niveau du cervelet et de la moëlle épinière ; par conséquent ils présentent une affinité pour les sites $\omega_1$ et $\omega_2$ (benzodiazépiniques de type 1 et de type 2) situés au sein du complexe macromoléculaire $GABA_A$-sites modulateurs w-canal chlorure. *In vivo* ils se comportent comme des agonistes complets ou partiels vis-à-vis de ces récepteurs.
Ils possèdent des propriétés hypnotiques, anxiolytiques et anticonvulsivantes et, par conséquent, peuvent être utilisés pour le traitement d'affections liées aux désordres de la transmission GABAergique, tels que l'anxiété, les troubles du sommeil, l'épilepsie, la spasticité, les contractures musculaires, les troubles cognitifs, les troubles du sevrage vis-à-vis de l'alcoolisme, etc.

A cet effet ils peuvent être présentés sous toutes formes galéniques, associés à des excipients appropriés, pour l'administration entérale ou parentérale, par exemple sous forme de comprimés, dragées, gélules, capsules, solutions ou suspensions buvables ou injectables, suppositoires, etc, dosés pour permettre une administration journalière de 1 à 1000 mg de substance active.

## Revendications

1. Composé répondant à la formule générale (I)

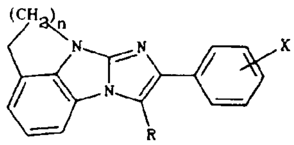

(I)

dans laquelle
n représente le nombre 1 ou 2,
X représente un atome d'hydrogène ou un ou deux atomes ou groupes choisis parmi le fluor, le chlore et les groupes alkyles en $C_1$-$C_3$, alcoxy en $C_1$-$C_3$ et hydroxy, et
R représente soit un atome d'hydrogène, soit un groupe de formule générale $-CH_2-CO_2-R_1$ (dans laquelle $R_1$ représente un atome d'hydrogène ou un groupe alkyle en $C_1$-$C_6$), soit un groupe de formule générale $-CH_2-CO-NR_2R_3$ (dans laquelle $R_2$ et $R_3$, indépendamment l'un de l'autre, représentent chacun un atome d'hydrogène ou un groupe alkyle en $C_1$-$C_3$),
à l'état de base ou de sel.

2. Composé selon la revendication 1, caractérisé en ce que
n représente le nombre 1,
X représente un atome d'halogène ou un groupe méthyle ou méthoxy, et
R représente un groupe de formule générale $-CH_2-CO-NH-CH_3$ ou $-CH_2-CO-N(CH_3)(CH_3)_2$.

3. Composé selon la revendication 1, à savoir le 8-(4-fluorophényl)-*N*-méthyl-4,5-dihydroimidazo[1,2-*a*]pyrrolo[1,2,3-*cd*]benzimidazole-9-acétamide.

4. Procédé de préparation de composés selon la revendication 1, caractérisé en ce qu'on fait réagir un dérivé de formule générale (II)

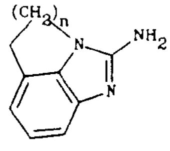

(II)

dans laquelle n est tel que défini dans la revendication 1, avec une 2-halo-1-phényléthanone de formule générale (III)

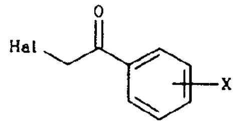

(III)

dans laquelle X est tel que défini dans la revendication 1 et Hal représente un atome de chlore ou de brome, pour obtenir un sel de guanidinium de formule générale (IV)

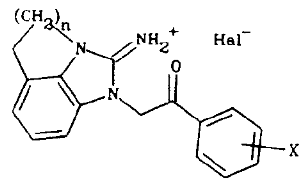

(IV)

que l'on cyclise pour obtenir un dérivé de formule générale (Ia)

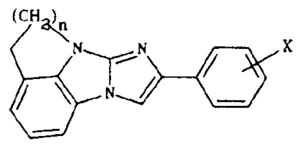

(Ia)

laquelle correspond à la formule générale (I) lorsque R représente H,
puis, si on le désire, on fait réagir ce dérivé de formule générale (Ia) avec le *N,N*-diméthylglyoxamide pour obtenir un dérivé d'α-hydroxyacétamide de formule générale (V)

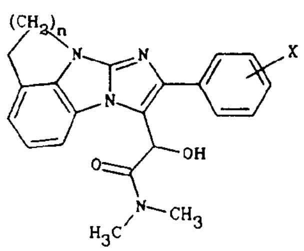

(V)

qu'on traite ensuite avec un polyhalogénure d'acide sulfurique ou phosphorique, pour former le dérivé de α-halogénoacétamide correspondant, puis on fait réagir ce dernier avec un agent réducteur pour obtenir un dérivé de *N,N*-diméthylacétamide de formule générale (Ib)

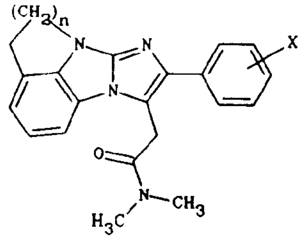

(Ib)

laquelle correspond à la formule générale (I) lorsque R représente $-CH_2-CO-N(CH_3)_2$,
puis, si on le désire, on transforme par hydrolyse le composé de formule générale (Ib) en acide de formule générale (Ic)

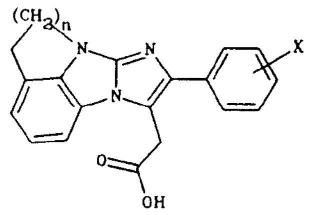

(Ic)

et finalement, si on le désire, on fait réagir l'acide de formule générale (Ic)

- soit avec le chlorure de thionyle, dans un solvant de type alcool aliphatique en $C_1$-$C_6$, pour obtenir un ester de formule générale (Id)

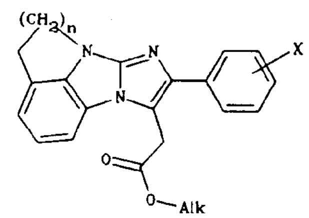

(Id)

dans laquelle Alk représente un groupe alkyle en $C_1$-$C_6$,

- soit avec le *N,N'*-carbonyldiimidazole, pour obtenir l'imidazolide correspondant et on traite ce dernier avec une amine de formule générale $HNR_2R_3$, pour obtenir un amide de formule générale (Ie)

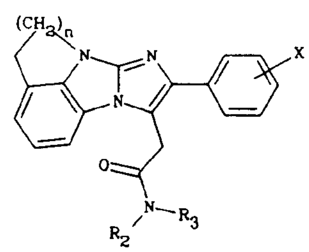

(Ie)

dans laquelle $R_2$ et $R_3$ sont tels que définis dans la revendication 1.

**5.** Médicament caractérisé en ce qu'il consiste en un composé selon l'une des revendications 1 à 3.

**6.** Composition pharmaceutique caractérisée en ce qu'elle contient un composé selon l'une des revendications 1 à 3, associé à un excipient.

Office européen des brevets

# RAPPORT DE RECHERCHE EUROPEENNE

Numero de la demande
EP 95 40 1014

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP-A-0 172 097 (SYNTHELABO) 19 Février 1986<br>* page 14, ligne 21 - ligne 32; revendication 1 *<br>--- | 1,6 | C07D471/16<br>C07D487/16<br>A61K31/435<br>A61K31/415 |
| A | WO-A-90 15058 (UPJOHN) 13 Décembre 1990<br>* page 2, ligne 14 - ligne 19; revendication 1 *<br>--- | 1,6 | //(C07D471/16, 235:00,235:00, 221:00), (C07D487/16, 235:00,235:00, 209:00) |
| A | FR-A-2 100 813 (SANKYO) 24 Mars 1972<br>* revendications 1,8 *<br>--- | 1,6 | |
| P,A | EP-A-0 607 076 (SYNTHELABO) 20 Juillet 1994<br>* page 25, ligne 56 - page 27, ligne 29 *<br>----- | 1,6 | |

DOMAINES TECHNIQUES RECHERCHES (Int.Cl.6)

C07D
A61K

Le présent rapport a été établi pour toutes les revendications

| Lieu de la recherche | Date d'achèvement de la recherche | Examinateur |
|---|---|---|
| LA HAYE | 27 Juin 1995 | Alfaro Faus, I |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)